(19) Europäisches Patentamt

European Patent Office

Office européen des brevets

(11) **EP 0 755 248 B1**

(12) **FASCICULE DE BREVET EUROPEEN**

(45) Date de publication et mention
de la délivrance du brevet:
**30.06.1999 Bulletin 1999/26**

(51) Int. Cl.$^6$: **A61K 9/127**, A61K 9/51,
A61K 9/16

(21) Numéro de dépôt: 95917383.2

(22) Date de dépôt: **12.04.1995**

(86) Numéro de dépôt international:
**PCT/FR95/00473**

(87) Numéro de publication internationale:
**WO 95/27477 (19.10.1995 Gazette 1995/45)**

(54) **MICROSPHERES GELIFIEES, LEUR PROCEDE DE PREPARATION ET LEURS APPLICATIONS**

GEL-MIKROSPHÄREN, VERFAHREN ZU DEREN HERSTELLUNG SOWIE IHRE
VERWENDUNGEN

GELIFIED MICROSPHERES, METHOD OF PREPARATION AND APPLICATIONS

(84) Etats contractants désignés:
**AT BE CH DE DK ES FR GB GR IE IT LI LU MC NL
PT SE**

(30) Priorité: **12.04.1994 FR 9404294**

(43) Date de publication de la demande:
**29.01.1997 Bulletin 1997/05**

(73) Titulaire: **Lipogel
13718 Allauch Cédex (FR)**

(72) Inventeurs:
• **HAUTON, Jacques
F-13012 Marseille (FR)**
• **SALLES, Jean-Pierre
F-13510 Eguilles (FR)**

(74) Mandataire: **Orès, Bernard et al
Cabinet ORES
6, Avenue de Messine
75008 Paris (FR)**

(56) Documents cités:
EP-A- 0 277 776          WO-A-89/02267
WO-A-91/00084            US-A- 4 740 375

**Description**

La présente invention est relative à des microsphères gélifiées, à leur procédé de préparation et à leurs applications.

Les microsphères, qui sont des particules de forme sphérique, dont la taille s'échelonne, généralement, entre 1 et 1 250 µm, sont constituées d'un matériau support contenant la substance encapsulée et présentent un intérêt particulier, soit lorsqu'il est souhaitable d'administrer un médicament sous une forme permettant la libération contrôlée du composant actif au cours d'une certaine période, afin d'assurer une action pharmacologique prolongée, soit lorsqu'il est nécessaire de protéger ledit composant actif d'une dégradation prématurée dans les voies digestives.

Suivant la structure du matériau de support, on distingue deux types de microcapsules :

- les microcapsules de type réservoir dans lesquelles le matériau support est une enveloppe solide, d'épaisseur variable contenant la substance à encapsuler,
- les microcapsules de type matriciel, appelées également microsphères, dans lesquelles le matériau support est un réseau continu, dans lequel est dispersée la substance à encapsuler.

Au sens de la présente invention, le terme microcapsule ou microsphère ne comprend que les microcapsules ou microsphères de type matriciel.

De nombreuses substances peuvent être encapsulées : il peut s'agir de produits chimiques tels que des médicaments, ou bien des macromolécules telles que des enzymes, et également des cellules vivantes.

Les microsphères sont utilisées dans de nombreux domaines, tels que la pharmacie, la bio-industrie, la cosmétologie, l'agroalimentaire, l'industrie papetière, etc...

Plusieurs procédés de préparation de microcapsules ont été décrits ; on peut notamment citer :

- la méthode par séparation de phase, notamment décrite dans le brevet US 4 675 189 et la Demande EP 52 510 qui décrit des microcapsules préparées par une technique de séparation de phase en utilisant un agent de coacervation, tel que les huiles minérales ou les huiles végétales.
   Cependant, les microcapsules préparées par cette méthode ainsi que par d'autres procédés analogues ont l'inconvénient de former des agglutinats (inter-adhésion de particules), lors de la préparation desdites microcapsules.
- la méthode par évaporation de solvant, notamment décrite dans le Brevet US 4 479 911, la Demande EP 301 969 et la Demande EP 145 240 : cette méthode comprend la formation séparée

   . d'une phase organique par dissolution d'un polymère convenable dans un solvant non miscible à l'eau et volatil et
   . d'une phase aqueuse contenant le principe actif intéressant,

   l'addition de la phase aqueuse dans la phase organique, le mélange des deux phases sous agitation et/ou en présence d'un agent émulsifiant puis l'évaporation du solvant, généralement sous agitation et à température ambiante, pour obtenir les microcapsules désirées.

La Demande EP 145 240 décrit plus particulièrement des microcapsules produites en préparant une émulsion E/H (émulsion primaire) comprenant une couche interne aqueuse contenant une substance hydrophile et une substance dite de rétention du médicament (mucilage naturel ou synthétique ou des composés de poids moléculaires élevés et plus particulièrement de la gélatine) et une couche huileuse contenant un polymère, de préférence un acide polylactique ou un copolymère d'acide lactique et d'acide glycolique ou leurs mélanges, dans un solvant non miscible à l'eau tel que le dichlorométhane, puis en épaississant ou en solidifiant ladite couche interne aqueuse de manière à obtenir une viscosité supérieure à 5 000 centipoises, puis en préparant une émulsion secondaire E/H/E, en présence d'un agent tensio-actif convenable, et finalement en soumettant l'émulsion ainsi obtenue à une évaporation du solvant. Le procédé décrit dans cette Demande permet l'obtention de microcapsules d'un diamètre compris entre 0,5 et 400 µm.

La Demande de Brevet européen n° 0 277 776 décrit un procédé de préparation de liposomes avec un noyau central solide comprenant les étapes de :

   (a) formation de prévésicules avec un noyau central solide encapsulé consistant essentiellement en une solution polymère-gel dans un solvant organique contenant un ou plusieurs émulsifiants, obtenu à partir d'une émulsion gélatine-agarose dispersée dans un solvant organique ;
   (b) extraction des composés lipophiles des prévésicules pour obtenir des microsphérules et notamment des microsphérules d'agarose-gélatine et,

(c) encapsulation des microsphérules dans des liposomes en utilisant la méthode d'évaporation de phase organique.

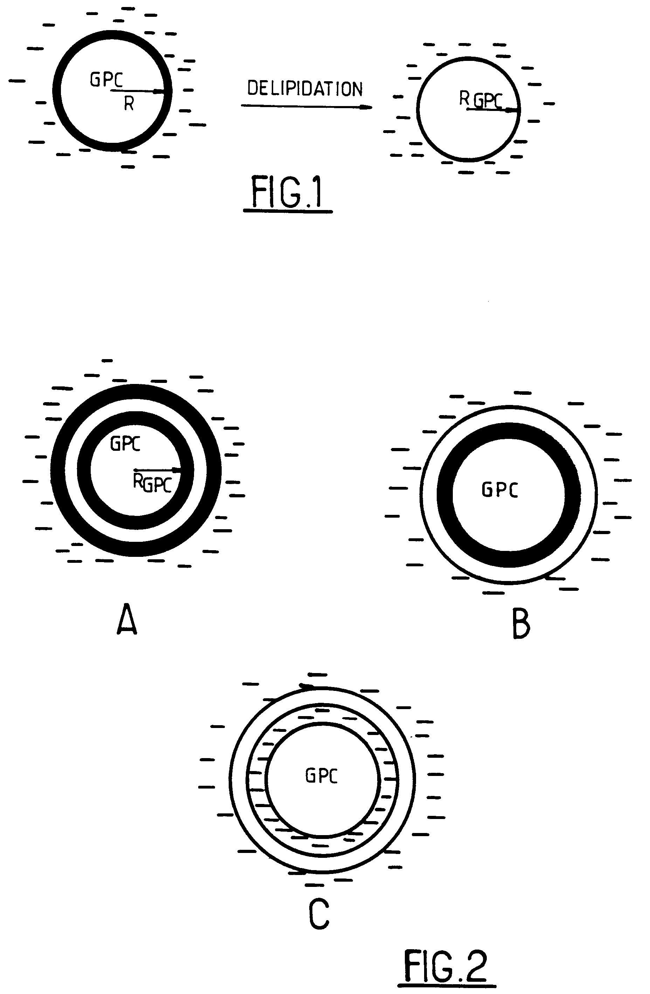

On obtient ainsi des liposomes contenant un noyau central formé d'un réseau agarose-gélatine (voir figure 1).

L'émulsification est difficile à obtenir et nécessite un mélange complexe comprenant des surfactifs aussi bien dans la phase aqueuse que dans la phase organique. Par exemple, à l'exemple 1, les prévésicules sont obtenues par mélange à 70°C d'une phase organique contenant de la lécithine d'oeuf, du cyclohexane, de l'éthanol et du span 80, dans une phase aqueuse contenant 5,33 % d'agarose et 2,67 % de gélatine, de la calcéine, une solution tampon, du N-octylglucoside et des billes de verre.

Toutefois ces microcapsules ou microsphères de l'Art antérieur ont l'inconvénient majeur d'avoir des diamètres de l'ordre du μm ou plus (1-1250 μm) ; or, il existe de nombreuses applications pour lesquelles il est nécessaire et/ou particulièrement avantageux de pouvoir disposer de particules ayant un diamètre significativement plus petit, notamment de l'ordre du nm ou plus, entre 20 et 600 nm, par exemple.

En conséquence, les Demanderesses se sont donné pour but la mise au point de microsphères gélifiées dont le diamètre peut être contrôlé et atteindre, si nécessaire, 20 nm.

La présente invention a pour objet des microsphères, caractérisées en ce qu'elles comprennent un noyau polaire gélifié (GPC = *gelified polar core*) autour duquel sont superposées concentriquement et alternativement n bicouches lipidiques ou n couches aqueuses à l'état liquide et n couches polaires gélifiées, n étant un nombre entier et en ce qu'elles sont susceptibles d'être obtenues par délipidation de liposomes, dénommés lipogélosomes[®] (marque déposée au nom de la société LIPOGEL et désignant des liposomes à noyau polaire gélifié), du type comportant au moins une bicouche lipidique externe et au moins une phase polaire aqueuse interne contenant une substance gélifiée.

De manière avantageuse, de telles microsphères :

- présentent un diamètre contrôlable, de préférence compris entre 20 et 600 nm,
- sont stables,
- peuvent inclure des substances actives hydrosolubles,
- permettent aussi bien la réalisation de formes à libération immédiate ou retard, selon le point de fusion de la substance gélifiée,
- peuvent servir de base à l'accrochage de ligands, de substances mal reconnues par le système réticulo-endothélial (microsphères furtives), de composés électriquement chargés (ciblage électrique en électrothérapie), et
- sont aptes à être rendues furtives (non reconnaissance par le système réticulo-endothélial), lorsqu'elles présentent un diamètre de l'ordre de 20-40 nm.

Conformément à l'invention, la substance gélifiable est sélectionnée parmi des composés gélifiables, polymérisables ou non, tels que les polysaccharides, les polypeptides ou les polyacrylamides.

De manière préférée, la substance gélifiable non polymérisable est sélectionnée parmi la gélatine, l'agarose, ou les carraghenanes et la substance gélifiable polymérisable est sélectionnée parmi les gels de polyacrylamide.

De tels liposomes à noyau polaire gélifié ou lipogélosomes sont notamment décrit dans le Brevet EP 0 393 049, dans lequel il est précisé qu'ils sont constitués par une phase interfaciale en bicouche dans le cas des lipogélosomes unilamellaires ou d'une pluralité de phases interfaciales en bicouche superposées concentriquement, dans le cas des lipogélosomes multilamellaires et par une phase polaire aqueuse interne encapsulée gélifiée.

Ce brevet décrit en particulier le procédé d'obtention de tels lipogélosomes unilamellaires ou multilamellaires : la phase aqueuse encapsulée gélifiée, est issue de la phase aqueuse initiale liquide, dans laquelle lesdits lipogélosomes sont préparés, par transformation de ladite phase aqueuse en gel, en raison de la présence, dans ladite phase aqueuse, d'un ou plusieurs composés gélifiables, polymérisables ou non ; la phase aqueuse non encapsulée peut, en outre être rendue ingélifiable par action physique, chimique ou enzymatique.

La/les phases interfaciales en bicouche sont constituées par exemple de lipides de classe 4 (phospholipides) éventuellement associés à des lipides de classe 2 et de classe 3 (cholestérol libre) et/ou des lipides de classe 5.

Cette classification des lipides proposée par le Professeur HAUTON et coll., basée sur les coefficients de partage $K_D$ entre une phase polaire aqueuse et une phase interfaciale en monocouche ou en bicouche et $K_C$ entre une phase interfaciale et une phase hydrophobe ou apolaire, sera utilisée (HAUTON et LAFONT, Biochimie, 1987, 69, 177-204) et est également décrite dans le Brevet EP 393 049 précité.

De tels lipogélosomes (LGS) sont généralement classés selon le nombre de bicouches :

. en petits et grands lipogélosomes unilamellaires : SULGS = *small unilamellar lipogelosomes* ; LULGS = *large unilamellar lipogelosomes* et

. en lipogélosomes multilamellaires : MLGS = *multilamellar lipogelosomes*.

Conformément à l'invention, l'étape de délipidation desdits lipogélosomes peut être mise en oeuvre de plusieurs manières et conduire, à des microsphères gélifiées de diamètre contrôlé :

a) délipidation superficielle des lipogélosomes unilamellaires ou multilamellaires par :

    (1) extraction de la bicouche lipidique superficielle desdits lipogélosomes unilamellaires ou multilamellaires, par un solvant organique ou un mélange de solvants organiques, non miscible à l'eau ;
    (2) partage biphasique de la phase organique et de la phase aqueuse ; et
    (3) séparation de la phase aqueuse contenant les microsphères gélifiées, superficiellement délipidées, (élimination de la bicouche la plus externe).

Dans le cas de la délipidation de lipogélosomes unilamellaires (élimination de l'unique bicouche lipidique), on obtient des microsphères gélifiées conformes à l'invention, également dénommées gélosomes® (GS) (marque déposée au nom de la société LIPOGEL) (petits gélosomes homogènes : SHGS = *small homogenous gelosomes* ; grands gélosomes homogènes : LHGS = *large homogenous gelosomes*) ou des polymérisomes® (marque déposée au nom de la société LIPOGEL et désignant également des microsphères gélifiées conformes à l'invention) homogènes, c'est-à-dire ne comportant aucune bicouche lipidique et donc constitués par des microsphères aqueuses gélifiées homogènes, correspondant au noyau polaire gélifié précité (GPC), polymérisé ou non.

Dans le cas de la délipidation superficielle de lipogélosomes multilamellaires (élimination de la bicouche lipidique la plus externe), on obtient des structures hybrides entre lipogélosome (LGS) et gélosome (GS) dénommées gélosomes (GS) multicouches hybrides (hybrid MGS = *hybrid multilayered gelosomes*) ou polymérisomes multicouches hybrides, constitués par un noyau polaire gélifié (GPC), polymérisé ou non, sur lequel se superposent concencentriquement des bicouches lipidiques, séparées par des couches aqueuses gélifiées, polymérisées ou non, la couche la plus externe étant une couche aqueuse gélifiée, polymérisée ou non.

De telles microsphères conformes à l'invention, représentent en fait :

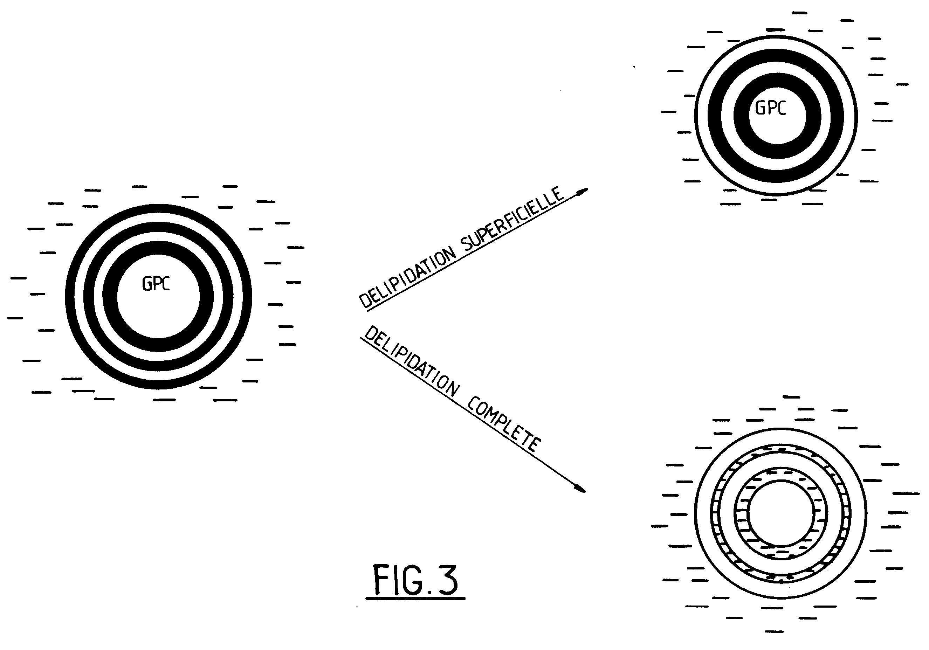

-   des LGS unilamellaires, lorsque les LGS initiaux non délipidés, qui ont servi à leur préparation, étaient bilamellaires, ou
-   des LGS multilamellaires, lorsque les LGS initiaux non délipidés, qui ont servi à leur préparation, étaient multilamellaires ;
   ces microsphères sont entourées par une couche aqueuse superficielle polymérisée ou non (Tableau I et figures 2 et 3).

Le solvant organique non miscible à l'eau est notamment, et ce de manière non limitative, de l'heptane.

b) délipidation complète des lipogélosomes unilamellaires ou multilamellaires par :

    (1) extraction lipidique des lipogélosomes unilamellaires ou multilamellaires, par un solvant organique ou un mélange de solvants organiques, miscible à l'eau ou partiellement miscible à l'eau ;
    (2) partage biphasique de la phase organique et de la phase aqueuse ;
    (3) séparation du/des solvant(s) organique(s) de la phase aqueuse ; et
    (4) séparation des microsphères gélifiées et complètement délipidées.

Conformément à l'invention, lorsque l'étape (1) est réalisée à l'aide d'une phase organique miscible à l'eau, un solvant organique apolaire est ajouté préalablement à l'étape (2), permettant ainsi le partage biphasique.

Dans le cas de la délipidation complète de lipogélosomes unilamellaires (élimination de l'unique bicouche lipidique), on obtient des gélosomes (GS) ou des polymérisomes homogènes, tels que définis ci-dessus.

Dans le cas de la délipidation complète de lipogélosomes bilamellaires, on obtient un noyau polaire gélifié (GPC), polymérisé ou non, entouré d'une couche aqueuse à l'état liquide et d'une seule couche superficielle aqueuse à l'état gélifié, polymérisée ou non.

Dans le cas de la délipidation complète de lipogélosomes multilamellaires, on obtient des gélosomes (GS) multicouches (MGS = *multilayered gelosomes*) ou polymérisomes multicouches, constitués par un noyau polaire gélifié (GPC), polymérisé ou non, sur lequel se superposent concencentriquement des couches aqueuses gélifiées, polymérisées ou non, séparées entre elles par des couches aqueuses à l'état liquide.

Le solvant organique est avantageusement mais de manière non limitative, du n-butanol.

La présente invention a également pour objet un procédé de préparation de microsphères conformes à l'invention, comprenant un noyau polaire gélifié (GPC = *gelified polar core*) autour duquel sont, éventuellement, superposées concentriquement et alternativement $\underline{n}$ bicouches lipidiques ou $\underline{n}$ couches aqueuses à l'état liquide et $\underline{n}$ couches polaires gélifiées, $\underline{n}$ étant un nombre entier, lequel procédé est caractérisé en ce qu'il comprend :

(a) la préparation de liposomes, dénommés lipogélosomes, du type comportant n+1 bicouches lipidiques, dont une bicouche lipidique externe et au moins une phase polaire aqueuse interne contenant une substance gélifiée, et
(b) la délipidation desdits lipogélosomes.

L'étape (a) est décrite dans le Brevet EP 0 393 049.

Selon un mode de mise en oeuvre avantageux dudit procédé, préalablement à l'étape de délipidation, les lipogélosomes sont sélectionnés en fonction de leur diamètre, de préférence par ultrasonication.

Selon un autre mode de mise en oeuvre avantageux dudit procédé, préalablement à l'étape de délipidation, les substances non encapsulées sont éliminées, de préférence par ultrafiltration tangentielle.

On obtient alors un concentré de lipogélosomes dans le rétentat d'ultrafiltration : l'étape de délipidation est alors effectuée dans ledit rétentat d'ultrafiltration.

Selon un autre mode de mise en oeuvre avantageux dudit procédé, l'étape (b) de délipidation comprend, pour une délipidation superficielle :

(1) l'extraction de la bicouche lipidique superficielle desdits lipogélosomes unilamellaires ou multilamellaires, par un solvant organique ou un mélange de solvants organiques, non miscible à l'eau ;
(2) le partage biphasique de la phase organique et de la phase aqueuse ; et
(3) la séparation de la phase aqueuse contenant les microsphères gélifiées, superficiellement délipidées, telles que définies ci-dessus.

Selon encore un autre mode de mise en oeuvre avantageux dudit procédé, l'étape (b) de délipidation comprend, pour une délipidation complète :

(1) l'extraction lipidique des lipogélosomes unilamellaires ou multilamellaires, par un solvant organique ou un mélange de solvants organiques, miscible à l'eau ou partiellement miscible à l'eau ;
(2) le partage biphasique de la phase organique et de la phase aqueuse ;
(3) l'élimination du solvant organique de la phase aqueuse ; et
(4) la séparation des microsphères gélifiées et complètement délipidées, telles que définies ci-dessus.

Outre les dispositions qui précèdent, l'invention comprend encore d'autres dispositions, qui ressortiront de la description qui va suivre, qui se réfère à des exemples de mise en oeuvre du procédé objet de la présente invention, ainsi qu'aux dessins annexés dans lesquels :

-    la figure 1 illustre une microsphère gélifiée obtenue par délipidation d'un lipogélosome unilamellaire :
-    la figure 2 illustre une microsphère gélifiée obtenue par délipidation superficielle ou complète d'un lipogélosome bilamellaire ;
-    la figure 3 illustre une microsphère gélifiée obtenue par délipidation superficielle ou complète d'un lipogélosome multilamellaire ;
-    la figure 4 illustre les variations des volumes molaires de lipogélosomes unilamellaires, en fonction du rayon R (coordonnées log/log) ;
-    la figure 5 illustre les variations des volumes molaires de gélosomes homogènes (SHGS ou LHGS), en fonction du rayon R-h (coordonnées log/log) ;
-    la figure 6 illustre les variations des volumes molaires de lipogélosomes multilamellaires, en fonction du rayon R (coordonnées log/log).

Il doit être bien entendu, toutefois, que ces exemples sont donnés uniquement à titre d'illustration de l'objet de l'invention, dont ils ne constituent en aucune manière une limitation.

Les différents types de microsphères gélifiées conformes à l'invention sont illustrés au Tableau I ci-après, ainsi qu'aux figures 1 à 3.

TABLEAU I

| | | délipidation superficielle | délipidation complète |
|---|---|---|---|
| LGS unilamellaires (SULGS et LULGS) | GPC non polymérisé | gélosomes homogènes (SHGS et LHGS) (figure 1) | gélosomes homogènes (SHGS et LHGS) (figure 1) |
| | GPC polymérisé | polymérisomes homogènes (figure 1) | polymérisomes homogènes (figure 1) |
| LGS multilamellaires (MLGS) | GPC non polymérisé | gélosomes multicouches hybrides : GPC + bicouches lipidiques concentriques séparées par couches aqueuses gélifiées (figures 2 et 3) | gélosomes multicouches : GPC + couches aqueuses gélifiées concentriques séparées par couches aqueuses à l'état liquide (figures 2 et 3) |
| | GPC polymérisé | polymérisomes multicouches hybrides : GPC + bicouches lipidiques concentriques séparées par couches aqueuses gélifiées (figures 2 et 3) | polymérisomes multicouches : GPC + couches aqueuses gélifiées concentriques séparées par couches aqueuses à l'état liquide (figures 2 et 3) |

La figure 1 illustre les microsphères gélifiées, polymérisées ou non, obtenues par délipidation superficielle ou complète d'un lipogélosome unilamellaire.

Les lipogélosomes unilamellaires (SULGS et LULGS) comprennent un noyau polaire gélifié (GPC), polymérisé ou non (représenté en blanc) de rayon $R_{GPC}$ et une bicouche de phospholipides (représentée en foncé) d'épaisseur h = $10^{-8}$ cm ; le rayon desdits lipogélosomes unilamellaires est $R = R_{GPC} + h$.

Par délipidation du lipogélosome unilamellaire, on obtient, comme précisé ci-dessus, un gélosome homogène (SHGS et LHGS), si le noyau polaire gélifié est non polymérisé, ou un polymérisome homogène, si le noyau polaire gélifié est polymérisé.

Le rayon de la microsphère gélifiée conforme à l'invention, polymérisée ou non, est égal à $R_{GPC}$.

La phase aqueuse liquide environnant les LGS et les microsphères est représentée par une série de traits (hachurage).

La figure 2 illustre les microsphères gélifiées, polymérisées ou non, obtenues par délipidation superficielle ou complète d'un lipogélosome bilamellaire.

La figure 2A représente un lipogélosome bilamellaire comprenant un noyau polaire gélifié (GPC), polymérisé ou non (représenté en blanc), de rayon $R_{GPC}$ et deux bicouches lipidiques (représentées en foncé), d'épaisseur h = $10^{-8}$ cm, et séparées par une couche aqueuse gélifiée d'épaisseur H ; le rayon desdits lipogélosomes (LGS) bilamellaires $R_{LGS} = R_{GPC} + 2h + H$. (1)

La figure 2B représente les gélosomes ou polymérisomes multicouches hybrides obtenus par délipidation superficielle desdits LGS bilamellaires : seule la bicouche lipidique superficielle étant extraite, on obtient des microsphères qui comprennent un noyau polaire gélifié, polymérisé ou non, et entouré d'une bicouche lipidique, puis d'une couche aqueuse gélifiée superficielle d'épaisseur H, polymérisée ou non.

Le rayon de ces microsphères conformes à l'invention est $R = R_{GPC} + h + H$. (2)

La figure 2C représente les gélosomes ou polymérisomes multicouches obtenus par délipidation complète desdits LGS bilamellaires : les deux bicouches lipidiques étant extraites, on obtient un gélosome ou un polymérisome non-homogène constitué par un noyau polaire gélifié (GPC), polymérisé ou non, une couche aqueuse à l'état liquide, d'épaisseur h, et enfin une couche aqueuse gélifiée superficielle, polymérisée ou non, d'épaisseur H ; le rayon de ces microsphères est $R = R_{GPC} + h + H$.

La figure 3 représente les gélosomes ou polymérisomes multicouches obtenus par délipidation superficielle ou délipidation complète de lipogélosomes multilamellaires (MLGS).

Ces MLGS comprennent un noyau polaire gélifié (GPC), polymérisé ou non (représenté en blanc), de rayon $R_{GPC}$ et n bicouches lipidiques (représentées en foncé), avec n = 3, l'épaisseur de chaque bicouche étant h = $10^{-8}$ cm ; le rayon desdits lipogélosomes multilamellaires $R_{LGS} = R_{GPC} + nh + (n-1)H$ (3), est de, lorsque n = 3, égal à $R_{GPC} + 3h + 2H$ (4).

Par délipidation superficielle desdits LGS trilamellaires, on obtient des gélosomes ou des polymérisomes multicouches hybrides comprenant un noyau polaire gélifié (GPC), polymérisé ou non, une bicouche lipidique, puis une première couche aqueuse gélifiée, une deuxième couche lipidique, puis superficiellement une deuxième couche aqueuse gélifiée, polymérisée ou non.

Le rayon de telles microsphères conformes à l'invention est $R = R_{GPC} + 2h + 2H$ (5).

Par délipidation complète desdits LGS trilamellaires, on obtient des gélosomes ou des polymérisomes multicouches comprenant un noyau polaire gélifié (GPC), polymérisé ou non, une première couche aqueuse à l'état liquide (représentée en hachurée), une première couche aqueuse gélifiée, une deuxième couche aqueuse à l'état liquide, puis superficiellement une deuxième couche aqueuse gélifiée, polymérisée ou non.

Le rayon de telles microsphères conformes à l'invention est $R = R_{GPC} + 2h + 2H$.

**EXEMPLE 1 : Détermination des paramètres physiques des lipogélosomes (LGS) et des microsphères conformes à l'invention (superficiellement délipidées ou complètement délipidées) : appréciation du contrôle du diamètre des microsphères.**

Une condition nécessaire pour obtenir une méthode standardisée de calcul des paramètres physiques d'un type bien défini de particules sphériques est de respecter strictement un système d'Unités LMT.

Parmi les différents systèmes d'Unités, l'ancien système C.G.S. est le mieux adapté aux habitudes des biologistes. Mais il est facile de passer d'un système d'Unités à l'autre par l'utilisation de facteurs de conversion appropriés.

Dans le respect du système C.G.S., il faut donc toujours exprimer une longueur en cm, une surface en $cm^2$, un volume en $cm^3$, une densité en $g.cm^{-3}$, une concentration en $g.cm^{-3}$ ou en $mol.cm^{-3}$ et un temps en seconde, conditions nécessaires pour pouvoir obtenir des modèles standardisés dont l'absence constitue une lacune dans les Sciences biologiques par l'absence d'utilisation d'Unités cohérentes entres elles (HAUTON J.C. et al., BIOCHIMIE, 1987, 69, 177-204, *Lipid Biodynamics: new perspectives*).

1) Paramètres physiques d'une espèce particulaire sphérique bien définie :

une particule sphérique X, telle qu'un lipogélosome (LGS), un gélosome (GS) ou une structure hybride entre lipogélosome (LGS) ou gélosome (GS), de rayon R en cm, est une entité plurimoléculaire de volume $v_X$ en $cm^3$, de densité $d_X$ en $g.cm^{-3}$, de masse $m_X$ en g et de surface $s_X$ en $cm^2$ (les paramètres physiques d'une particule étant exprimés par des caractères minuscules).

Ainsi, une mole de X, soit N particules de X ou $6,023x10^{23}$ particules (les paramètres physiques molaires étant exprimés par des caractères majuscules) a un volume molaire MV (*Molar Volume*) $MV_X$ de $v_X N$ $cm^3$, égal à $4/3\pi R^3 N$ $cm^3$ ou $25,22x10^{23}R^3$ $cm^3$, une masse molaire MM (*Molar Mass*) $MM_X$ de $m_X N$ g ou $25,22xR^3 d_X$ g et une surface molaire MS (*Molar Surface*) $MS_X$ de $s_X N$ $cm^2$, égale à $4\pi R^2 N$ $cm^2$ ou $75,68x10^{23}R^2$ $cm^2$ ($4/3\pi=4,188$ ; $4/3\pi N=25,23x10^{23}$ ; $4\pi=12,5664$ et $4\pi N=75,69x10^{23}$).

Si la concentration de l'espèce particulaire X est exprimée en $mol.cm^{-3}$ par [X], on a alors par $cm^3$ d'un volume de référence bien défini (phase aqueuse, *in vitro*, sang total, plasma, fluide interstitiel, bile, contenu intraluminaire, etc...), la relation suivante donnant le nombre $n_X$ de particules par $cm^3$ :

$$n_X = [X]N \text{ ou } n_X = [X]/N^{-1}$$

(l'inverse du nombre d'AVOGADRO $N^{-1}$ égal à $0,166x10^{-23}$ devient une constante exprimant en $mol.cm^{-3}$ la concentration d'un seul atome, d'une seule molécule ou d'une seule particule). Un paramètre physique ou chimique molaire d'une espèce particulaire multiplié par N ou divisé par $N^{-1}$ donne la valeur de ce paramètre par particule.

2) Délipidation des lipogélosomes (LGS) unilamellaires :

Dans le cas des lipogélosomes (LGS) unilamellaires, d'un rayon donné R en cm, on a une phase aqueuse encapsulée gélifiée, polymérisée ou non, dite PP (*Polar Phase*) se présentant sous la forme d'une sphère de rayon (R-h) cm, h exprimant l'épaisseur en cm de la bicouche lipidique superficielle, dite BIP (*Bilayer Interfacial Phase*). La sphère aqueuse gélifiée encapsulée est désignée par le symbole GPC (*Gelified Polar Core*).

Les résultats sont obtenues à partir de la méthode de calcul exposée ci-dessus, et montrent les caractéristiques physiques des lipogélosomes (LGS) unilamellaires de divers diamètres, et des microsphères conformes à l'invention, c'est-à-dire gélifiées et obtenues par délipidation (soit les GPC). Les symboles suivants sont utilisés :

R = rayon donné des lipogélosomes (LGS) en cm,

h = épaisseur en cm d'une bicouche lipidique superficielle (BIP) avoisinant $40x10^{-8}$cm (soit 40 Å ou 4 nm). Une demi-BIP avoisine donc $20x10^{-8}$cm.

$MV_{LGS}$ = volume molaire en $cm^3$ des lipogélosomes (LGS) d'un rayon donné R.

$MV_{GPC}$ = volume molaire en $cm^3$ du noyau polaire gélifié (GPC) de lipogélosomes (LGS) unilamellaires d'un rayon donné R, soit le volume molaire des microsphères obtenues par la délipidation des lipogélosomes (LGS) unilamellaires.

$MV_{BIP}$ = volume molaire en $cm^3$ de l'unique BIP superficielle entourant les lipogélosomes (LGS) unilamellaires d'un rayon donné R.

$MV_{BIP(e)}$ = volume molaire de la monocouche phospholipidique externe de l'unique BIP superficielle entourant les lipogélosomes (LGS) unilamellaires d'un rayon donné R.

$MV_{BIP(i)}$ = volume molaire de la monocouche phospholipidique interne de l'unique BIP superficielle entourant les LGS unilamellaires d'un rayon donné R.

$MS_{LGS}$ = surface molaire en $cm^2$ de lipogélosomes (LGS) d'un rayon donné R.

$MS_{GPC}$ = surface molaire en $cm^2$ des GPC de rayon (R-h)cm, soit la surface molaire des microsphères gélifiées obtenues par délipidation.

Quatre exemples sont montrés.

a) Lipogélosomes (LGS) unilamellaires de R= 100 x $10^{-8}$ cm (soit diamètre de 20 nm) :

$MV_{LGS}$ = 2,55 x $10^6$ $cm^3$,
$MV_{GPC}$ = 0,54 x $10^6$ $cm^3$ (soit 22 M de $MV_{LGS}$),
$MV_{BIP}$ = 1,98 x $10^6$ $cm^3$ (soit 78 % de $MV_{LGS}$),
$MV_{BIP(e)}$ = 1,23 x $10^6$ $cm^3$ (soit 62,2 % de $MV_{BIP}$),
$MV_{BIP(i)}$ = 0,75 x $10^6$ $cm^3$ (soit 37,8 % de $MV_{BIP}$),
$MS_{LGS}$ = 7,57 x $10^{12}$ $cm^2$,
$MS_{GPC}$ = 2,72 x $10^{12}$ $cm^2$.

b) Lipogélosomes (LGS) unilamellaires de R= 500 x $10^{-8}$ cm (soit diamètre de 100 nm) :

$MV_{LGS}$ = 315 x $10^6$ $cm^3$,
$MV_{GPC}$ = 247 x $10^6$ $cm^3$ (soit 77,9 % de $MV_{LGS}$),
$MV_{BIP}$ = 70 x $10^6$ $cm^3$ (soit 22,1 % de $MV_{LGS}$),
$MV_{BIP(e)}$ = 36 x $10^6$ $cm^3$ (soit 52 % de $MV_{BIP}$),
$MV_{BIP(i)}$ = 33 x $10^6$ $cm^3$ (soit 48 % de $MV_{BIP}$),
$MS_{LGS}$ = 189 x $10^{12}$ $cm^2$,
$MS_{GPC}$ = 160 x $10^{12}$ $cm^2$.

c) Lipogélosomes (LGS) unilamellaires de R = 2 500 x $10^{-8}$ cm (soit diamètre de 500 nm) :

$MV_{LGS}$ = 39 413 x $10^6$ $cm^3$,
$MV_{GPC}$ = 37 545 x $10^6$ $cm^3$ (soit 95,3 % de $MV_{LGS}$),
$MV_{BIP}$ = 1 862 x $10^6$ $cm^3$ (soit 4,7 % de $MV_{LGS}$),
$MV_{BIP(e)}$ = 939 x $10^6$ $cm^3$ (soit 50,4 % de $MV_{BIP}$),
$MV_{BIP(i)}$ = 923 x $10^6$ $cm^3$ (soit 49,6 % de $MV_{BIP}$),
$MS_{LGS}$ = 4 730 x $10^{12}$ $cm^2$,
$MS_{GPC}$ = 4 580 x $10^{12}$ $cm^2$.

d) Lipogélosomes (LGS) unilamellaires de R = 10 000 x $10^{-8}$ cm (soit diamètre de 2 000 nm ou 2 microns) :

$MV_{LGS}$ = 2 522 432 x $10^6$ $cm^3$,
$MV_{GPC}$ = 2 492 284 x $10^6$ $cm^3$ (soit 98,8 % de $MV_{LGS}$),
$MV_{BIP}$ = 30 155 x $10^6$ $cm^3$ (soit 1,2 % de $MV_{LGS}$),
$MV_{BIP(e)}$ = 15 108 x $10^6$ $cm^3$ (soit 50,1 % de $MV_{BIP}$),
$MV_{BIP(i)}$ = 15 047 x $10^6$ $cm^3$ (soit 49,9 % de $MV_{BIP}$),
$MS_{LGS}$ = 75 690 x $10^{12}$ $cm^2$,
$MS_{GPC}$ = 75 076 x $10^{12}$ $cm^2$.

Par délipidation des petits (SULGS) ou des grands (LULGS) lipogélosomes unilamellaires, l'unique bicouche lipidique superficielle est extraite ; seuls les GPC (*Gelified Polar Core*) subsistent, donnant ainsi des gélosomes (GS) homogènes, polymérisés (*homogenous polymerisomes*) ou non polymérisés, comme précisé ci-dessus.

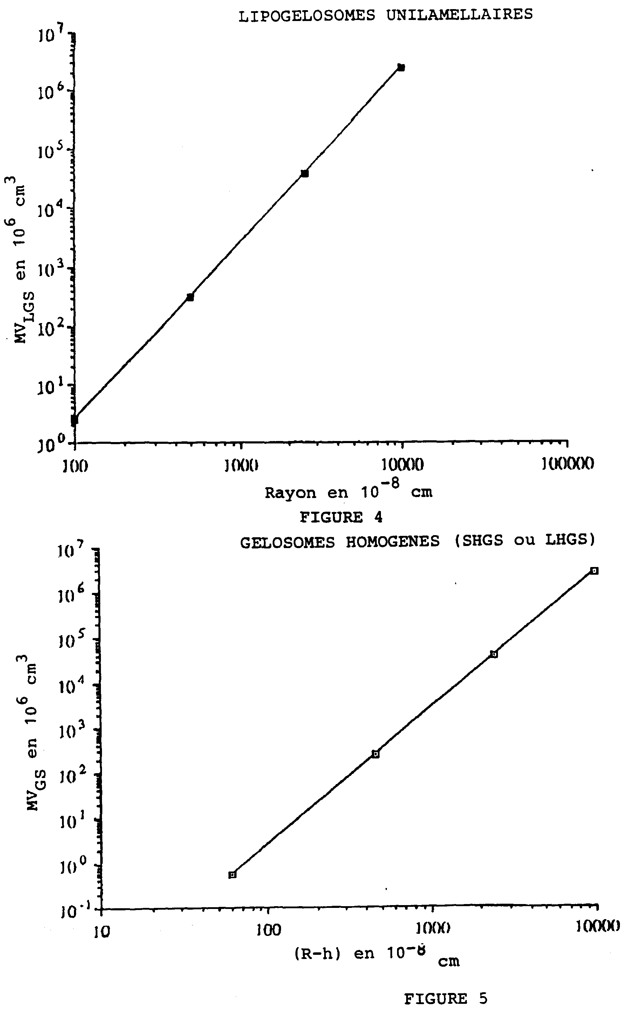

Les figures 4 et 5 montrent en coordonnées log/log, d'une part les variations des $MV_{LGS}$ en $cm^3$ en fonction

du rayon R exprimé en cm et d'autre part celles des $MV_{GS}$ en fonction du rayon (R-h) exprimé en cm.

Les résultats exprimés en MV (*Molar Volume*) en $cm^3.mol^{-1}$ peuvent s'exprimer en MM (*Molar Mass*) en $g.mol^{-1}$ en multipliant le MV par la densité d en $g.cm^{-3}$. Connaissant la densité $d_{pp}$ de la phase polaire aqueuse encapsulée gélifiée, polymérisée ou non, et celle de la bicouche lipidique superficielle $d_{BIP}$, il est alors possible de calculer la densité $d_{LGS}$ des lipogélosomes (LGS) ou celle $d_{GS}$ des gélosomes (GS).

3) Délipidation de lipogélosomes (LGS) multilamellaires :

Les lipogélosomes (LGS) multilamellaires (MLGS) consistent en un GPC (*Gelified Polar Core*) polymérisé ou non, d'un rayon $R_{GPC}$ exprimé en cm, sur lequel se superposent concentriquement une première bicouche lipidique d'épaisseur h avoisinant $40x10^{-8}$ cm, puis une couche aqueuse gélifiée, polymérisée ou non, d'épaisseur H de l'ordre de $100 \times 10^{-8}$ cm, puis d'une deuxième bicouche lipidique, et ainsi de suite.

Le symbole n représentant le nombre de bicouches lipidiques, le rayon R d'un lipogélosome multilamellaire (MLGS) est donné par l'équation (3) ci-dessus.

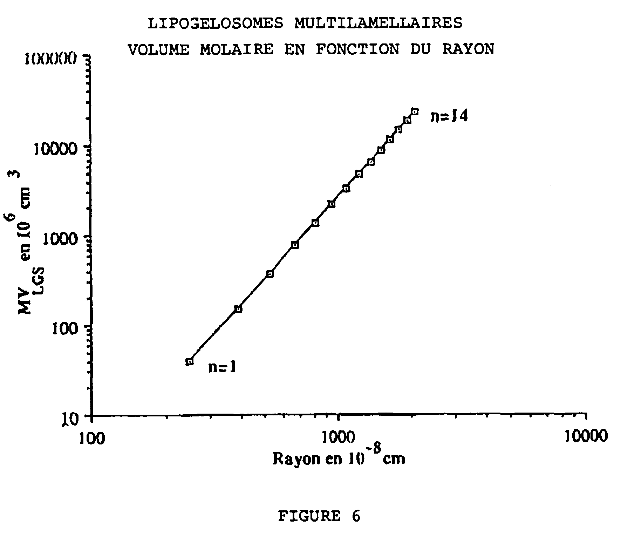

Le Tableau II montre comme exemple les variations des $MV_{LGS}$ et les pourcentages volumiques respectifs $\%MV_{pp}$ de la phase aqueuse encapsulée gélifiée, polymérisée ou non, et $\%MV_{BIP}$ de la phase lipidique de LGS ayant un $R_{GPC}$ de $210 \times 10^{-8}$ cm et n bicouches lipidiques, et donc (n-1) couches aqueuses gélifiées, polymérisées ou non, d'épaisseur $100 \times 10^{-8}$ cm. La figure 6 montre en coordonnées log/log les paramètres précités, pour des lipogélosomes multilamellaires. En pratique, il faut déterminer les paramètres $R_{GPC}$, n, h et H d'une espèce définie de lipogélosome multilamellaire (MLGS) pour bien préciser leurs caractéristiques physiques.

TABLEAU II

| Nombre de BIP n | R $10^{-8}$ cm | $MV_{LGS}$ $10^6 cm^3.mol^{-1}$ | % $MV_{PP}$ | % $MV_{BIP}$ |
|---|---|---|---|---|
| 1 | 250 | 39 | 59.3 | 40.7 |
| 2 | 390 | 150 | 61.8 | 38.2 |
| 3 | 530 | 370 | 63.7 | 36.8 |
| 4 | 670 | 760 | 65.2 | 34.8 |
| 5 | 810 | 1 340 | 66.2 | 33.8 |
| 6 | 950 | 2 160 | 67 | 33.0 |
| 7 | 1090 | 3 270 | 67.5 | 32.5 |
| 8 | 1230 | 4 690 | 68 | 32.0 |
| 9 | 1370 | 6 480 | 68.3 | 31.7 |
| 10 | 1510 | 8 680 | 68.6 | 41.4 |
| 11 | 1650 | 11 300 | 68.8 | 31.2 |
| 12 | 1790 | 14 500 | 69.0 | 31.0 |
| 13 | 1930 | 18 100 | 69.2 | 30.8 |
| 14 | 2070 | 22 400 | 69.3 | 30.7 |

Conformément à l'invention, lors de la délipidation superficielle, seule la bicouche lipidique superficielle ou externe d'épaisseur h des lipogélosomes multilamellaires (MLGS) est extraite. On obtient alors des structures hybrides entre lipogélosomes (LGS) et gélosomes (GS) comprenant une couche aqueuse gélifiée d'épaisseur H, polymérisée ou non, et contenant (n-1) bicouches lipidiques entourant le GPC, polymérisé ou non. Le rayon de ces structures hybrides sera égal à $R_{LGS}$-h, $R_{LGS}$ étant le rayon des lipogélosomes multilamellaires (MLGS) avant délipidation.

Conformément à l'invention, lors de la délipidation complète, toutes les bicouches lipidiques des lipogélosomes multilamellaires (MLGS) sont extraites, laissant un espace qui sera occupé par la phase polaire aqueuse à l'état liquide lors du partage phase organique/phase aqueuse. On obtient ainsi les structures non homogènes désignées comme gélosomes multicouches (MGS) (*multilayered gelosomes*), dont la phase aqueuse encapsulée gélifiée initiale provenant des lipogélosomes multilamellaires (MLGS) est polymérisée (*multilayered polymerisomes*) ou non. Le rayon de ces gélosomes (GS) non homogènes sera égal $R_{LGS}$-h, $R_{LGS}$ étant le rayon des lipogélosomes multilamellaires (MLGS) avant délipidation. Après délipidation complète, le pourcentage molaire de l'espace lipidique ainsi libéré et occupé par la phase aqueuse liquide provenant du partage solvant/eau sera égal au % $MV_{BIP}$ des lipogélosomes multilamellaires (MLGS) non délipidés.

**EXEMPLE 2 : Préparation de microsphères conformes à l'invention.**

A. Produits :

a) phospholipides :
La phase lipidique est constituée par des phospholipides de soja déshuilé STERN-France, présenté sous forme sèche et est utilisée à une concentration de 7,5 % en poids/volume.
b) Agents gélifiables non polymérisables :

- la gélatine, de type B150 Blooms Sanofi-Bioindustries, France ; elle a une température de fusion entre 30 et 35°C et est utilisée à une concentration de 7,5 % en poids/volume.
- le carraghénane, de Sanofi-Bioindustries, France. Le mélange gélatine/carraghénane à 80/20 (p/p) a une température de fusion de 50°C. Ce mélange est utilisé à une concentration de 7,5 % en poids/volume.
  La gélatine ou le mélange gélatine/carraghénane, dans les proportions et concentrations précitées, donne une phase aqueuse gélifiée non polymérisée au dessous des températures de fusion respectives de 30-35°C et 50°C.

c) Agent gélifiable polymérisable :

- Acrylamide/bis-acrylamide SIGMA.
  Une phase aqueuse gélifiée polymérisée est obtenue par mélange d'acrylamide/bis-acrylamide SIGMA, de Temed[®] BIO-RAD et de persulfate d'ammonium BIO-RAD, dans des conditions de concentrations appropriées ; une concentration de 15 % en poids/volume en acrylamide/bis-acrylamide est notamment utilisée.
  Le gel de polyacrylamide constitue un exemple non limitatif, sélectionné parmi les composés polymérisables.

d) Agent cryoprotecteur :

- saccharose SIGMA (utilisé à 7,5 % en poids/volume).

B. Protocole opératoire :
Les concentrations données sont celles utilisées dans la phase aqueuse initiale, dans laquelle sont dispersés les phospholipides.
Parmi les divers procédés utilisés pour la préparation des liposomes et des lipogélosomes, et adaptables à l'échelle industrielle, le procédé suivant est préférable, car il comporte une étape d'ultrasonication (adaptée à l'échelle industrielle) permettant d'obtenir une grande prépondérance de lipogélosomes (LGS) d'un diamètre de l'ordre de 100 nm, soit un rayon R de 500 x $10^{-8}$ cm. Par diffusion quasi-élastique mesuré par un granulomètre laser de Sema-Tech (Nice), 81 % de la population en nombre des lipogélosomes (LGS) sont centrés sur un diamètre de 92,7 nm (un rayon R de 413 x $10^{-8}$ cm) et 19 % sur un diamètre de 312 nm (un rayon R de 1 560 x $10^{-8}$ cm).
Ce procédé comprend :

1) Agitation mécanique lente de phospholipides de soja déshuilé STERN-France à une concentration de 7,5 % (p/v) pendant 3 heures, dispersés dans une phase aqueuse contenant l'agent gélifiant (7,5 % de gélatine ou du mélange gélatine/carraghénane, ou 15 % d'acrylamide/bis-acrylamide sans Temed[®] et persulfate d'ammonium), à l'état liquide. Eventuellement du saccharose à 7,5 % (p/v) est ajouté comme agent cryoprotecteur.

a) pour les agents gélifiants non polymérisables (gélatine ou mélange gélatine/carraghénase), cette étape d'agitation mécanique s'effectue au dessus du point de fusion de ces agents gélifiants.
b) pour le polyacrylamide comme agent gélifiant polymérisable, cette agitation mécanique s'effectue sans addition de Temed[®] et de persulfate d'ammonium, ce qui empêche le processus de polymérisation.
Cette étape d'agitation mécanique produit des liposomes multilamellaires (puisque non encore transformés en lipogélosomes) d'un diamètre variant de 297 à 2 084 nm avec un diamètre moyen de 504 nm (soit un rayon R de 2 520 x $10^{-8}$ cm).

2) Ultrasonication par un ultrasonicateur SONROREATOR (Undatim Ultrasonics, Louvain-La-Neuve, Belgique) avec sonotrodes en titane adaptés au volume de la suspension de liposomes. La fréquence de 20 KHz est utilisée, la puissance étant adaptée au volume de la suspension de liposomes. Pour des volumes de l'ordre de 10 $cm^3$, le temps de sonication se situe entre 3 et 4 minutes, tandis qu'il est élevé à 10 minutes pour des volumes de 700 à 750 $cm^3$.

a) Pour les agents gélifiants non polymérisables précités, la sonication se fait au dessus du point de fusion de ces agents.

b) Pour l'agent gélifiant polymérisable polyacrylamide, le Temed$^{®}$ et le persulfate d'ammonium induisant le processus de polymérisation sont ajoutés en début de l'ultrasonication. Une dilution aqueuse immédiate en fin de sonication (1/10 à 1/20e) empêche la polymérisation de la phase aqueuse non encapsulée dans les liposomes, tandis que la phase aqueuse encapsulée dans ceux-ci va polymériser.

3) Elimination des composés non encapsulés par ultrafiltration tangentielle, les liposomes et/ou lipogélosomes restant dans le rétentat. Le matériel utilisé provenant de Filtron (France) peut être dimensionné en fonction du volume à traiter. Les membranes utilisées ont un seuil de coupure de 300 kDa ou 1 000 kDa (soit plus correctement de 300 000 ou 1 million de g.$mol^{-1}$).

En l'absence d'une dilution aqueuse, l'ultrafiltration tangentielle des liposomes doit se faire à une température supérieure à la température de fusion des agents gélifiants non polymérisables (gélatine ou mélange gélatone/carraghénane). Une dilution aqueuse appropriée après la sonication peut permettre d'utiliser l'ultrafiltration tangentielle à la température ambiante. Pour augmenter le rendement de la dialyse des composés gélifiés encapsulés non polymérisables, la papaïne a été utilisée pour fragmenter la gélatine non encapsulée. Par la suite, l'abaissement de la température va transformer les liposomes en lipogélosomes qui vont se concentrer dans le rétentat.

Pour le polyacrylamide comme agent gélifiant polymérisable, une dilution appropriée et une ultrafiltration suivant immédiatement la sonication élimine l'agent polymérisable non encapsulé, tandis que l'agent polymérisable encapsulé va transformer les liposomes en lipogélosomes.

4) Délipidation superficielle ou complète des lipogélosomes (LGS) :

Les lipogélosomes (LGS) plus ou moins concentrés dans le rétentat d'ultrafiltration, selon les conditions opératoires choisies, sont superficiellement délipidés par un solvant (ou un mélange de solvants) non miscible à l'eau. L'heptane a été utilisé comme solvant non miscible à l'eau. Une délipidation complète s'obtient en utilisant un solvant miscible à l'eau, ou de préférence partiellement miscible à l'eau, tel que le n-butanol. Après un partage biphasique phase organique/phase aqueuse, les diverses microsphères précédemment décrites sont récupérées dans la phase polaire aqueuse, soit : petits (SHGS) ou grands (LHGS) gélosomes homogènes non polymérisés, petits (SHGS) ou grands (LHGS) gélosomes homogènes polymérisés (ou *homogenous polymerisomes*), lipogélosomes multilamellaires (MLGS) entourés par une couche aqueuse gélifiée non polymérisée (structures hybrides entre lipogélosomes (LGS) et gélosomes (GS), lipogélosomes multilamellaires (MLGS) entourés par une couche aqueuse gélifiée polymérisée (structures hybrides entre lipogélosomes (LGS) et gélosomes (GS), et gélosomes multicouches non polymérisés ou polymérisés (*multilayered polymerisomes*).

Par granulométrie laser et microscopie électronique, un contrôle de la diminution des diamètres obtenus par délipidation superficielle ou complète des lipogélosomes (LGS) unilamellaires permet de vérifier que le procédé utilisé s'est effectué correctement. Par ailleurs, le dosage des lipides dans la phase organique obtenue après le partage solvant(s)/eau permet de vérifier l'efficacité de la délipidation.

## Revendications

1. Microsphères, caractérisées en ce qu'elles comprennent un noyau polaire gélifié autour duquel sont superposées concentriquement et alternativement n bicouches lipidiques ou n couches aqueuses à l'état liquide et n couches polaires gélifiées, n étant un nombre entier, égal ou supérieur à 1, et en ce qu'elles sont susceptibles d'être obtenues par délipidation de liposomes, du type comportant au moins une bicouche lipidique externe et au moins une phase polaire aqueuse interne contenant une substance gélifiée, dénommés liposomes à noyau polaire gélifié.

2. Microsphères selon la revendication 1, caractérisées en ce que la substance gélifiable est sélectionnée parmi des composés gélifiables, polymérisables ou non, tels que les polysaccharides, les polypeptides ou les polyacrylamides.

3. Microsphères selon la revendication 2, caractérisées en ce que la substance gélifiable non polymérisable est sélectionnée parmi la gélatine, l'agarose, ou les carraghenanes et la substance gélifiable polymérisable est sélectionnée parmi les gels de polyacrylamide.

4. Microsphères selon l'une quelconque des revendications 1 à 3, caractérisées en ce qu'elles présentent un diamètre compris entre 20 et 600 nm.

5. Microsphères selon l'une quelconque des revendications 1 à 4, caractérisées en ce que la délipidation est mise en oeuvre par délipidation superficielle des liposomes à noyau polaire gélifié unilamellaires ou multilamellaires par :

(1) extraction de la bicouche lipidique superficielle desdits liposomes à noyau polaire gélifié unilamellaires ou multilamellaires, par un solvant organique ou un mélange de solvants organiques, non miscible à l'eau ;
(2) partage biphasique de la phase organique et de la phase aqueuse ; et
(3) séparation de la phase aqueuse contenant les microsphères gélifiées, superficiellement délipidées.

6. Microsphères selon l'une quelconque des revendications 1 à 4, caractérisées en ce que la délipidation est mise en oeuvre par délipidation complète des liposomes à noyau polaire gélifié unilamellaires ou multilamellaires par :

(1) extraction lipidique des liposomes à noyau polaire gélifié unilamellaires ou multilamellaires, par un solvant organique ou un mélange de solvants organiques, miscible à l'eau ou partiellement miscible à l'eau ;
(2) partage biphasique de la phase organique et de la phase aqueuse ;
(3) séparation du/des solvant(s) organique(s) de la phase aqueuse ; et
(4) séparation des microsphères gélifiées et complètement délipidées.

7. Microsphères selon la revendication 6, caractérisées en ce que, lorsque l'étape (1) est réalisée à l'aide d'une phase organique miscible à l'eau, un solvant organique apolaire est ajouté préalablement à l'étape (2).

8. Procédé de préparation de microsphères selon l'une quelconque des revendications 1 à 7, comprenant un noyau polaire gélifié autour duquel sont superposées concentriquement et alternativement n bicouches lipidiques ou n couches aqueuses à l'état liquide et n couches polaires gélifiées, n étant un nombre entier, lequel procédé est caractérisé en ce qu'il comprend :

(a) la préparation de liposomes à noyau polaire gélifié, du type comportant n+1 bicouches lipidiques, dont une bicouche lipidique externe et au moins une phase polaire aqueuse interne contenant une substance gélifiée, et
(b) la délipidation desdits liposomes à noyau polaire gélifié.

9. Procédé selon la revendication 8, caractérisé en ce que préalablement à l'étape de délipidation, les liposomes à noyau polaire gélifié sont sélectionnés en fonction de leur diamètre, de préférence par ultrasonication.

10. Procédé selon la revendication 8 ou la revendication 9, caractérisé en ce que, préalablement à l'étape de délipidation, les substances non encapsulées sont éliminées, de préférence par ultrafiltration tangentielle.

11. Procédé selon l'une quelconque des revendications 8 à 10, caractérisé en ce que l'étape (b) de délipidation comprend, pour une délipidation superficielle :

(1) l'extraction de la bicouche lipidique superficielle desdits liposomes à noyau polaire gélifié unilamellaires ou multilamellaires, par un solvant organique ou un mélange de solvants organiques, non miscible à l'eau ;
(2) le partage biphasique de la phase organique et de la phase aqueuse ;
(3) la séparation de la phase aqueuse contenant les microsphères gélifiées, superficiellement délipidées.

12. Procédé selon l'une quelconque des revendications 8 à 10, caractérisé en ce que l'étape (b) de délipidation comprend, pour une délipidation complète :

(1) l'extraction lipidique des liposomes à noyau polaire gélifié unilamellaires ou multilamellaires, par un solvant organique ou un mélange de solvants organiques, miscible à l'eau ou partiellement miscible à l'eau ;
(2) le partage biphasique de la phase organique et de la phase aqueuse ;
(3) la séparation du/des solvant(s) organique(s) de la phase aqueuse ; et
(4) la séparation des microsphères gélifiées et complètement délipidées.

**13.** Application des microsphères selon l'une quelconque des revendications 1 à 7, à la préparation d'un médicament apte à servir de vecteur de substances actives.

**14.** Application des microsphères selon l'une quelconque des revendications 1 à 7, à la préparation d'un réactif de diagnostic.

**15.** Application de microsphères constituées d'un noyau polaire gélifié, susceptibles d'être obtenues par délipidation de liposomes comportant au moins une bicouche lipidique externe et au moins une phase polaire aqueuse interne contenant une substance gélifiée, dénommés liposomes à noyau polaire gélifié, ladite substance gélifiée étant sélectionnée parmi des composés gélifiables, polymérisables ou non, tels que les polysaccharides, les polypeptides ou les polyacrylamides, les composés gélifiables non polymérisables étant sélectionnés parmi la gélatine, l'agarose ou les carraghénanes, à la préparation d'un médicament apte à servir de vecteur de substances actives.

## Claims

**1.** Microspheres, characterized in that they comprise a gelified polar core around which are superimposed, concentrically and alternately, $n$ lipid bilayers or $n$ aqueous layers in the liquid state and $n$ gelified polar layers, $n$ being an integer, equal or greater than 1, and in that they are capable of being obtained by delipidation of liposomes of the type containing at least one outer lipid bilayer and at least one inner polar aqueous phase containing a gelified substance, called liposomes with a gelified polar core.

**2.** Microspheres according to Claim 1, characterized in that the gelifiable substance is selected from polymerizable or non-polymerizable gelifiable compounds, such as polysaccharides, polypeptides or polyacrylamides.

**3.** Microspheres according to Claim 2, characterized in that the non-polymerizable gelifiable substance is selected from gelatin, agarose or carragheenins and the polymerizable gelifiable substance is selected from polyacrylamide gels.

**4.** Microspheres according to any one of Claims 1 to 3, characterized in that they exhibit a diameter of between 20 and 600 nm.

**5.** Microspheres according to any one of Claims 1 to 4, characterized in that the delipidation is carried out by surface delipidation of unilamellar or multilamellar liposomes with a gelified polar core by:

(1) extraction of the surface lipid bilayer of the said unilamellar or multilamellar liposomes with a gelified polar core by a water-immiscible organic solvent or a mixture of water-immiscible organic solvents;
(2) two-phase partition of the organic phase and of the aqueous phase; and
(3) separation of the aqueous phase containing the surface-delipidated gelified microspheres.

**6.** Microspheres according to any one of Claims 1 to 4, characterized in that the delipidation is carried out by complete delipidation of unilamellar or multilamellar liposomes with a gelified polar core by:

(1) lipid extraction of unilamellar or multilamellar liposomes with a gelified polar core by a water-miscible or partially water-miscible organic solvent or a mixture of water-miscible or partially water-miscible organic solvents;
(2) two-phase partition of the organic phase and of the aqueous phase;
(3) separation of the organic solvent or solvents from the aqueous phase; and
(4) separation of the gelified and completely delipidated microspheres.

**7.** Microspheres according to Claim 6, characterized in that, when the stage (1) is carried out using a water-miscible organic phase, a non-polar organic solvent is added prior to the stage (2).

**8.** Method for the preparation of microspheres according to any one of Claims 1 to 7 comprising a gelified polar core around which are superimposed, concentrically and alternately, $n$ lipid bilayers or $n$ aqueous layers in the liquid state and $n$ gelified polar layers, $n$ being an integer, which method is characterized in that it comprises:

(a) the preparation of liposomes with a gelified polar core of the type containing n+1 lipid bilayers, including an outer lipid bilayer, and at least one inner polar aqueous phase containing a gelified substance, and
(b) the delipidation of the said liposomes with a gelified polar core.

9. Method according to Claim 8, characterized in that, prior to the delipidation stage, the liposomes with a gelified polar core are selected according to their diameter, preferably by ultrasonication.

10. Method according to Claim 8 or Claim 9, characterized in that, prior to the delipidation stage, the non-encapsulated substances are removed, preferably by tangential ultrafiltration.

11. Method according to any one of Claims 8 to 10, characterized in that the delipidation stage (b) comprises, for surface delipidation:

(1) the extraction of the surface lipid bilayer of the said unilamellar or multilamellar liposomes with a gelified polar core by a water-immiscible organic solvent or a mixture of water-immiscible organic solvents;
(2) the two-phase partition of the organic phase and of the aqueous phase;
(3) the separation of the aqueous phase containing the surface-delipidated gelified microspheres.

12. Method according to any one of Claims 8 to 10, characterized in that the delipidation stage (b) comprises, for complete delipidation:

(1) the lipid extraction of unilamellar or multilamellar liposomes with a gelified polar core by a water-miscible or partially water-miscible organic solvent or a mixture of water-miscible or partially water-miscible organic solvents;
(2) the two-phase partition of the organic phase and of the aqueous phase;
(3) the separation of the organic solvent or solvents from the aqueous phase; and
(4) the separation of the gelified and completely delipidated microspheres.

13. Application of the microspheres according to any one of Claims 1 to 7 for the preparation of a medicament able to be used as a vehicle for active substances.

14. Application of the microspheres according to any one of Claims 1 to 7 for the preparation of a diagnostic reagent.

15. Application of microspheres constituted by a gelified polar core, capable of being obtained by delipidation of liposomes comprising at least one outer lipid bilayer and at least one inner polar aqueous phase containing a gelified substance, called liposomes with a gelified polar core, said gelifiable substance being selected from polymerizable or non-polymerizable gelifiable compounds, such as polysaccharides, polypeptides or polyacrylamides, the non-polymerizable gelifiable compounds being selected from gelatin, agarose or carragheenins, for the preparation of a medicament able to be used as a vehicle for active substances.

**Patentansprüche**

1. Mikrokügelchen, dadurch **gekennzeichnet,** daß sie einen polaren gelartigen Kern umfassen, um den konzentrisch und alternierend n Lipid-Doppelschichten, n wäßrige Schichten im flüssigen Zustand und n polare gelartige Schichten angeordnet sind, wobei n eine ganze Zahl von gleich oder größer 1 ist, und daß sie durch Delipidierung von Liposomen des Typs, der mindestens eine externe Lipid-Doppelschicht und mindestens eine interne polare wäßrige Phase, die eine gelartige Substanz enthält, umfaßt, mit der Bezeichnung Liposomen mit polarem gelartigem Kern, erhalten werden können.

2. Mikrokügelchen nach Anspruch 1, dadurch **gekennzeichnet,** daß die gelierbare Substanz aus gelierbaren polymerisierbaren oder nichtpolymerisierbaren Verbindungen wie Polysacchariden, Polypeptiden oder Polyacrylamiden ausgewählt ist.

3. Mikrokügelchen nach Anspruch 2, dadurch **gekennzeichnet,** daß die gelierbare nicht polymerisierbare Substanz aus Gelatine, Agarose oder Carragheenanen und die gelierbare polymerisierbare Substanz aus Polyacrylamidgelen ausgewählt ist.

4. Mikrokügelchen nach einem der Ansprüche 1 bis 3, dadurch **gekennzeichnet,** daß sie einen Durchmesser zwischen 20 und 600 nm besitzen.

5. Mikrokügelchen nach einem der Ansprüche 1 bis 4, dadurch **gekennzeichnet,** daß die Delipidierung durch oberflächliche Delipidierung von unilamellären oder mulitlamellären Liposomen mit einem polaren gelartigen Kern durchgeführt wird durch:

(1) Extraktion der oberflächlichen Lipid-Doppelschicht der unilamellären oder multilamellären Liposomen mit polarem gelartigem Kern durch ein organisches Lösungsmittel oder ein Gemisch von organischen Lösungsmitteln, das mit Wasser nicht mischbar ist;

(2) biphasische Verteilung der organischen Phase und der wäßrigen Phase; und

(3) Abtrennen der wäßrigen Phase, die die gelartigen oberflächlich delipidierten Mikrokügelchen enthält.

**6.** Mikrokügelchen nach einem der Ansprüche 1 bis 4, dadurch **gekennzeichnet,** daß die Delipidierung durch vollständige Delipidierung der unilamellären oder multilamellären Liposomen mit einem gelartigen polaren Kern durchgeführt wird durch:

(1) Lipidextraktion von unilamellären oder multilamellären Liposomen mit einem gelartigen polaren Kern durch ein organisches Lösungsmittel oder ein Gemisch von organischen Lösungsmitteln, das mit Wasser mischbar oder teilweise mischbar ist;

(2) biphasische Verteilung der organischen Phase und der wäßrigen Phase;

(3) Trennung des/der organischen Lösungsmittel(s) und der wäßrigen Phase; und

(4) Abtrennen der gelartigen und vollständig delipidierten Mikrokügelchen.

**7.** Mikrokügelchen nach Anspruch 6, dadurch **gekennzeichnet,** daß wenn die Stufe (1) mit Hilfe mit einer mit Wasser mischbaren organischen Phase durchgeführt wird, ein apolares organisches Lösungmittel vor der Stufe (2) zugesetzt wird.

**8.** Verfahren zur Herstellung von Mikrokügelchen nach einem der Ansprüche 1 bis 7, umfassend einen polaren gelartigen Kern, um den konzentrisch und alternativ n Lipid-Doppelschichten oder n wäßrige Schichten in flüssigem Zustand und n gelartige polare Schichten angeordnet sind, wobei n eine ganze Zahl ist, dadurch **gekennzeichnet,** daß es umfaßt:

(a) Herstellung der Liposomen mit einem polaren gelartigen Kern des Typs, der n+1 Lipid-Doppelschichten enthält, wovon eine externe Lipid-Doppelschicht und mindestens eine interne wäßrige polare Phase eine gelartige Substanz enthält, und

(b) Delipidierung der Liposomen mit gelartigem polarem Kern.

**9.** Verfahren nach Anspruch 8, dadurch **gekennzeichnet,** daß vor der Stufe der Delipidierung die Liposomen mit einem gelartigen polaren Kern als Funktion ihres Durchmessers, bevorzugt durch Ultraschall, ausgewählt werden.

**10.** Verfahren nach Anspruch 8 oder 9, dadurch **gekennzeichnet,** daß vor der Stufe der Delipidierung die nichtverkapselten Substanzen bevorzugt durch tangentiale Ultrafiltration entfernt werden.

**11.** Verfahren nach einem der Ansprüche 8 bis 10, dadurch **gekennzeichnet,** daß die Stufe (b) der Delipidierung für eine oberflächliche Delipidierung umfaßt:

(1) Extraktion der oberflächlichen Lipid-Doppelschicht der unilamellären oder multilamellären Liposomen mit einem polaren gelartigen Kern mit einem organischen Lösungsmittel oder einem Gemisch von organischen Lösungsmitteln, das mit Wasser nicht mischbar ist;

(2) biphasische Verteilung der organischen Phase und der wäßrigen Phase,

(3) Abtrennung der wäßrigen Phase, die die oberflächlich delipidierten gelartigen Mikrokügelchen enthält.

**12.** Verfahren nach einem der Ansprüche 8 bis 10, dadurch **gekennzeichnet,** daß die Stufe (b) der Delipidierung für eine vollständige Delipidierung umfaßt:

(1) Lipidextraktion der unilamellären oder multilamellären Liposomen mit einem polaren gelartigen Kern mit einem organischen Lösungsmittel oder einem Gemisch von organischen Lösungsmitteln, das mit Wasser mischbar oder teilweise mischbar ist;

(2) biphasische Verteilung der organischen Phase und der wäßrigen Phase;

(3) Abtrennung des/der organischen Lösungsmittel(s) von der wäßrigen Phase; und

(4) Trennung der gelartigen und vollständig delipidierten Mikrokügelchen.

**13.** Verwendung von Mikrokügelchen nach einem der Ansprüche 1 bis 7 zur Herstellung eines Medikaments, das sich dazu eignet, als Vektor für aktive Substanzen zu dienen.

**14.** Verwendung von Mikroktigelchen nach einem der Ansprüche 1 bis 7 zur Herstellung eines diagnostischen Reagenses.

**15.** Verwendung von Mikrokügelchen, bestehend aus einem polaren gelartigen Kern, die durch Delipidierung von Liposomen, die mindestens eine externe Lipid-Doppelschicht und mindestens eine interne polare wäßrige Phase, die eine gelartige Substanz enthält, umfassen, mit der Bezeichnung Liposomen mit gelartigem polarem Kern, wobei die gelartige Substanz aus gelierbaren polymerisierbaren oder nichtpolymerisierbaren Verbindungen, wie Polysacchariden, Polypeptiden oder Polyacrylamiden, ausgewählt wird, wobei die gelierbaren nichtpolymerisierbaren Verbindungen aus Gelatine, Agarose oder Carragheenanen ausgewählt werden, zur Herstellung eines Medikaments, das sich dazu eignet, als Vektor für aktive Substanzen zu dienen.

FIG.1

A

B

C

FIG.2

FIG.3

LIPOGELOSOMES UNILAMELLAIRES

FIGURE 4

GELOSOMES HOMOGENES (SHGS ou LHGS)

FIGURE 5

LIPOGELOSOMES MULTILAMELLAIRES
VOLUME MOLAIRE EN FONCTION DU RAYON

FIGURE 6